# EUROPÄISCHE PATENTANMELDUNG

(11) **EP 4 368 167 A1**
(43) Veröffentlichungstag der Anmeldung: **15.05.2024**
(21) Anmeldenummer: 22206261.4
(22) Anmeldetag: 08.11.2022
(51) Int. Cl.: A61K 6/60, A61K 6/62, A61K 6/76, A61K 6/887

(54) **DENTALWERKSTOFF ZUR HERSTELLUNG DENTALER VOLL- ODER TEILPROTHESEN**

(71) Anmelder: Ivoclar Vivadent AG, 9494 Schaan (LI)
(72) Erfinder: Haydl, Christoph, 9424 Rheineck (CH); Loiseau, Francois, 3254 Messen (CH); Häfele, Clemens Andreas, 6800 Feldkirch (AT); Dellafior, Petra, 6800 Feldkirch (AT)
(74) Vertreter: Uexküll & Stolberg

(57) **Zusammenfassung**

Radikalisch polymerisierbarer Werkstoff zur Herstellung von Dentalprothesen, der (a) mindestens ein monofunktionelles radikalisch polymerisierbares (Meth)acrylat, (b) mindestens ein polyfunktionelles radikalisch polymerisierbares (Meth)acrylat, (c) mindestens ein ABA-Triblockcopolymer, (d) mindestens einen nanopartikulären oder mikrofeinen Füllstoff mit einer mittleren Primärpartikelgröße vom 10 nm bis 350 nm, und (e) mindestens einen Photoinitiator enthält. Der A-Block des ABA-Triblockcopolymers ist ein oligomerer Polyester und der B-Block ein oligomeres Polysiloxan.

## Beschreibung

Die vorliegende Erfindung betrifft radikalisch polymerisierbare Dentalwerkstoffe die sich zur Herstellung der Prothesenbasis und der Ersatzzähne dentaler Voll- oder Teilprothesen eignen. Die Werkstoffe eignen sich besonders zur Verarbeitung mit 3D-Druckverfahren.

Dentale Voll- und Teilprothesen dienen als Ersatz für einen oder mehrere fehlende Zähne und bestehen aus der Prothesenbasis und den Ersatzzähnen. Die Prothesenbasis liegt zumindest teilweise an oder auf der Mundschleimhaut auf und trägt die Ersatzzähne. Voll- und Teilprothesen sind in der Regel abnehmbar. Vollprothesen werden auch als Totalprothesen und Teilprothesen als partielle Prothesen bezeichnet.

Die mechanischen Anforderungen an Prothesenbasis und Ersatzzähne unterscheiden sich erheblich, so dass zur Herstellung von Prothesenbasis und Ersatzzähnen unterschiedliche Werkstoffe verwendet werden. Dies ist mit einer Reihe von Nachteilen verbunden. Ein Problem ist die Schaffung eines festen Verbundes zwischen den Materialien, was bei unterschiedlichen Werkstoffen eine größere Herausforderung als bei einheitlichen Materialien ist. Ein weiteres Problem liegt darin, dass unterschiedliche Werkstoffe meist unterschiedliche Verarbeitungsverfahren erfordern und so die Herstellung von Prothesen erschweren. Zur Herstellung von Prothesen werden können additive Fertigungsverfahren eingesetzt werden, beispielsweise die Stereolithographie und das Inkjet-Drucken. Bei additiven Fertigungsverfahren, die auch als generative Fertigungsverfahren bezeichnet werden, werden 3D-Formkörper ausgehend von einem CAD-Datensatz schichtenweise aus polymerisierbaren Materialien erzeugt, wobei das Härten der Schichten durch gesteuerte Belichtung erfolgt. Inkjet-Druckverfahren bieten gegenüber der Stereolithographie den Vorteil, dass mehrere Werkstoffe in demselben additiven Verfahren verarbeitet werden können. Dies setzt jedoch eine gewisse Ähnlichkeit und Verträglichkeit der Werkstoffe voraus, so dass diese mit einer Apparatur gemeinsam verarbeitet werden können.

Ein weiteres Problem liegt im weltweiten Trend zur stetigen Verschärfung regulatorischen Anforderungen bei der Zulassung neuer Materialien für medizinische Anwendungen. Unter diesem Gesichtspunkt sind einheitliche Werkstoffe, die eine geringe Anzahl chemisch unterschiedlicher Komponenten enthalten, vorteilhaft, weil sie die Zulassung vereinfachen.

Die EP 2 492 289 A1 offenbart Dentalwerkstoffe, die Blockcopolymere, radikalisch polymerisierbare Monomere und einen Polymerisationsinitiator enthalten. Die Blockcopolymere enthalten harte Segmente aus Methacrylsäureestern und weiche Blöcke aus Acrylsäureestern. Die harten Blöcke zeichnen sich durch eine hohe und die weichen Blöcke durch eine geringe Glasübergangstemperatur aus. Die Materialien sind relativ flexibel, so dass sie bei Beanspruchung nicht brechen, weisen jedoch ein niedriges Biege-E-Modul auf. Sie sollen sich besonders als dentale Zemente eignen.

Die WO 2014/078537 A1 offenbart Harzmischungen zur Herstellung dentaler Formkörper durch 3D-Druckverfahren auf der Basis von mono- und multifunktionellen Methacrylaten, die Silicon-Acrylat-basierende Schlagzähmodifikatoren mit Kern-Schale-Struktur zur Verbesserung der Schlag- und Bruchzähigkeit erhalten. Diese Silicon-Acrylat-basierenden Modifikatoren neigen zur Verfärbung und schwimmen auf dem Monomergemisch auf bzw. sedimentieren, was nachteilig ist.

Die US 2018/0000570 A1 betrifft Baumaterialien auf der Basis von mono- und multifunktionellen (Meth)acrylaten zur generativen Herstellung dentaler Bauteile. Die Baumaterialien enthalten Gummipartikel auf Silikon-Acryl-Basis mit Kern-Schale-Struktur (Produkt S2006, Mitsubishi Rayon Co.) als Schlagzähmodifikatoren und Oligomere, die durch Umsetzung von Trimethyl-1,6-diiso-cyanat, Bisphenol-A-propoxylat und 2-Hydroxyethylmethacrylat (HEMA) hergestellt werden. Die gehärteten Bauteile sollen gute mechanische und physikalische Eigenschaften sowie eine gute Bioverträglichkeit aufweisen.

Die US 10,299,896 B2 und die US 2019/0053883 A1 offenbaren durch generative Verfahren hergestellte dentale Bauteile, die mindestens zwei Schichten aus unterschiedlich zusammengesetzten Baumaterialien aufweisen. Dabei wird eine Schicht durch ein Material gebildet, das Oligomere, die durch Reaktion von Zwischenprodukten mit endständigen Isocyanatgruppen mit hydroxylbasierten Methacrylaten erhalten werden, eine polymerisierbare Acrylverbindung und einen Schlagzähmodifikator enthält. Mindestens eine weitere Schicht wird durch ein Material gebildet, das ein Urethanmonomer, ein Glycoldimethacrylat und Füllstoff enthält. Die Kombination von Materialien mit unterschiedlichen mechanischen und physikalischen Eigenschaften soll zur Anpassung der Bauteile an unterschiedliche Anforderungen vorteilhaft sein. Als Schlagzähmodifikatoren werden handelsübliche Polymere mit Kern-Schale-Struktur verwendet, wie z.B. das Produkt M570 der Firma Kaneka.

Die EP 3 564 282 A1 offenbart härtbare Zusammensetzungen für Hochtemperatur-Photopolymerisationsverfahren, die ein oligomeres Urethandimethacrylat als Glasübergangstemperaturmodifikator, ein (Poly-)Carbonat-(Poly-)-Urethan-Dimethacrylat als Zähigkeitsmodifikator und ggf. Core-Shell-Partikel enthalten. Sie sollen gute thermomechanische Eigenschaften und eine gute Bioverträglichkeit aufweisen und sich zur Herstellung kieferorthopädischer Vorrichtungen eignen.

Die US 2020/0231803A1 offenbart härtbare Zusammensetzungen mit mindestens einem monofunktionellen Acrylat, einem multifunktionellen Acrylat und PPG-PEG-PPG oder PPG-PEG-PPG-Blockcopolymer-Acrylat, einem multifunktionellen Acrylat und PPG-PEG-PPG oder PPG-PEG-PPG-Blockcopolymer.

US 11,028,204 B2 offenbart radikalisch polymerisierbare Zusammensetzungen, die mindestens ein (Meth)acrylatmonomer oder -oligomer und mindestens ein monofunktionelles (Meth)acrylatmonomer mit einer polycyclischen Gruppe enthalten, die mindestens drei Ringe aufweist. Die Zusammensetzungen sollen sich durch hohe Zähigkeit, Zugfestigkeit und Zugdehnung auszeichnen und sich unter anderem als Tinten für den dreidimensionalen Inkjet-Druck für die Stereolithographie eignen.

Die bisher bekannten Werkstoffe sind nur bedingt zur Herstellung von Dentalprothesen, d.h. von Prothesenbasis und Ersatzzähnen, geeignet. Sie machen Zugeständnisse hinsichtlich der Eigenschaften der Prothesenbasis oder der Ersatzzähne erforderlich oder stellen einen Kompromiss dar, der für keinen der Prothesenbestandteile optimal ist.

Der Erfindung liegt die Aufgabe zugrunde, radikalisch polymerisierbare Werkstoffe zur Verfügung zu stellen, die sich sowohl zur Herstellung der Prothesenbasis als auch der Ersatzzähne eignen. Die Werkstoffe sollen sich durch generative Verfahren, insbesondere durch Stereolithographie und ganz besonders durch Inkjet-Druckverfahren verarbeiten lassen.

Erfindungsgemäß wird diese Aufgabe durch radikalisch polymerisierbare Werkstoffe gelöst, die
(a) mindestens ein monofunktionelles radikalisch polymerisierbares (Meth)acrylat, vorzugsweise Acrylat,
(b) mindestens ein polyfunktionelles radikalisch polymerisierbares (Meth)acrylat, vorzugsweise Methacrylat,
(c) mindestens ein Blockcopolymer,
(d) mindestens einen nanopartikulären oder mikrofeinen Füllstoff mit einer mittleren Primärpartikelgröße vom 10 nm bis 350 nm, und
(e) mindestens einen Photoinitiator enthalten.

Die erfindungsgemäßen Dentalwerkstoffe zeichnen sich dadurch aus, dass sie als Blockcopolymer (c) mindestens ein ABA-Triblockcopolymer enthalten.

Unter Blockcopolymeren werden Makromoleküle verstanden, die aus zwei oder mehr kovalent miteinander verbundenen Homopolymerblöcken bestehen. Die Werkstoffe sind vorzugsweise dadurch gekennzeichnet, dass der A-Block des ABA-Triblockcopolymers ein oligomerer Polyester, insbesondere ein oligomeres Polycaprolacton (PCL), und der B-Block ein oligomeres Polysiloxan, insbesondere ein PDMS, ist.

Erfindungsgemäß bevorzugte Blockcopolymere lassen sich mit den bekannten Methoden der lebenden bzw. kontrollierten Polymerisation herstellen, beispielsweise durch radikalische oder ionische (anionische und kationische) Polymerisation, wobei die kontrollierte radikalische Polymerisation und die lebende anionische Polymerisation bevorzugt sind. Blockcopolymere können aber auch durch die Verknüpfung von Homopolymeren über geeignete Endgruppen erhalten werden.

ABA-Blockcopolymere lassen sich beispielsweise herstellen, indem durch anionische Polymerisation von Monomer B über einen Dianionen-Mechanismus ein B-Block hergestellt wird. Der gebildete B-Mittelblock trägt beidseitig jeweils eine Anion-Endgruppe, die die anionische Polymerisation von Monomer A unter Bildung der beiden A-Blöcke initiiert (Methode 1). Alternativ können durch Veresterung eines telechelen B-Blocks, der an beiden Enden jeweils eine geeignete funktionelle Gruppe, z.B. eine OH-Gruppe trägt, mit zwei A-Blöcken, die nur einseitig, z.B. mit einer COOH-Gruppe, funktionalisiert sind, ABA-Triblockcopolymere erhalten werden (Methode 2). Schließlich lassen sich OH-telechele Homopolymere von Monomer B mit α-Bromisobuttersäure verestern. Die beiden so gebildeten Brom-Endgruppen im Homopolymerblock B lassen sich dann als Startzentrum für die Bildung der beiden A-Blöcke durch ATRP nutzen (Methode 3).

Die Monomere zur Herstellung der Blockcopolymere werden vorzugsweise so gewählt, dass die A-Blöcke mit der Harzmatrix, d.h. der Mischung der Bestandteile (a) und (b), mischbar und der B-Block nicht homogen mit der Harzmatrix mischbar ist.

Die Mischbarkeit wird hier im Sinne der Thermodynamik in Bezug auf die Einphasigkeit verstanden. Danach wird unter einem mischbaren Polymerblock ein Polymerblock aus einem Monomer verstanden, dessen Homopolymer in der Harz-Matrix löslich ist, so dass die Mischung eine Transparenz von mindestens 95 % aufweist. Ist die Mischung dagegen trüb oder opak, d.h. ist die Transparenz kleiner als 95%, so ist das Homopolymer und damit der entsprechende Polymerblock nicht mit der Harz-Matrix mischbar. Die Transparenz wird mit einem Spektrophotometer an 1 mm dicken, auf Hochglanz polierten Prüfkörpern in Transmission (D65) gemäß der Norm ISO 10526:1999 gemessen, z.B. mit einem Konika-Minolta Spektrophotometer des Typs CM-5.

Die Blockcoplymere bewirken eine deutliche Verbesserung der Bruchzähigkeit der erfindungsgemäßen Werkstoffe nach der Härtung. Es wird angenommen, dass die Unmischbarkeit der B-Blöcke der Blockcopolymere mit den übrigen Bestandteilen der erfindungsgemäßen Zusammensetzungen eine Mikrophasenseparation und damit die Ausbildung von Morphologien auf nanoskaliger Ebene bewirkt. Dabei bilden die Makromoleküle der ABA-Blockcopolymere im Monomerharz oder während der Aushärtung durch Selbstaggregation (self-assembly) sphärische oder wurmartige Phasen aus, die mit Rissspitzen interagieren können, d.h., dass Rissspitzen auf die Phasen treffen und sich die Bruchenergie dabei so in den Phasen verteilt, dass die Risse nicht weiter durch den Werkstoff wandern und sich nicht vergrößern. Die Fortpflanzung eines Risses lässt sich bei transparenten Materialen unter einem Elektronenmikroskop beobachten. In der Bruchmechanik wird der vorderste Teil des Risses als Rissspitze bezeichnet.

In einer bevorzugten Ausführungsform enthält der erfindungsgemäße Dentalwerkstoff als Komponente (c) ein siliziumhaltiges ABA-Triblockcopolymer.

ABA-Triblockcopolymere sind im Sinne der vorliegenden Erfindung "siliziumhaltig", wenn sie mindestens ein Si-Atom im Grundgerüst oder in der Seitenkette, vorzugsweise im Grundgerüst, aufweisen. Vorzugsweise enthält mindestens ein Block ein Polysiloxan-Oligomer. Ganz besonders bevorzugt ist der B-Block siliziumhaltig und insbesondere ein Polysiloxan-Oligomer.

Der A-Block ist vorzugsweise ein Polyester-Oligomer oder ein Poly(meth)acrylat. Ein besonders bevorzugtes Poly(meth)acrylat ist Polymethylmethacrylat (PMMA). Ein besonders bevorzugtes Beispiel für ein Polyester-Oligomer ist ein Polylacton-Oligomer und ganz bevorzugt ein Polymerisat von Caprolacton. Der A-Block ist damit vorzugsweise ein Polycaprolacton (PCL)-Oligomer.

Wenn der A-Block ein Poly(meth)acrylat ist, weist der A-Block eine Molmasse von 0,7 bis 7 kDa, vorzugsweise 1 bis 5,5 kDa und besonders bevorzugt 1,5 bis 4 kDa auf. In einer weiteren bevorzugten Ausführungsform ist der A-Block ein Poly(meth)acrylat und die Molmasse des gesamten ABA-Blockcopolymers beträgt weniger als 10 kDa, insbesondere weniger als 7 kDa.

Der B-Block ist vorzugsweise ein Polyolefin, insbesondere ein Polyethylen, oder ein Polysiloxan-Oligomer, besonders bevorzugt ein Polysiloxan-Oligomer gemäß der Formel -O-(SiR₂-O)ₚ, in der
- R: eine lineare C₁-C₂₀-Alkyl-, verzweigte C₃-C₁₂-Alkyl- oder C₆-C₂₀-Arylgruppe ist, wobei die einzelnen R-Reste gleich oder verschieden sein können, und
- p: eine Zahl von 3 bis 100, bevorzugt eine Zahl von 10 bis 50 ist.

Ganz besonders bevorzugt ist der B-Block ein Polymerisat von Dimethylchlorsilan, Cyclotri- oder Cyclotetradimethoxysilan. Der B-Block ist ganz besonders bevorzugt ein Poly(dimethylsiloxan) (PDMS)-Oligomer.

Die B-Blöcke zeichnen sich durch eine relativ hohe Flexibilität aus. Unter flexiblen Blöcken werden Blöcke verstanden, die aus Monomeren gebildet werden, deren Homopolymeren eine Glasübergangstemperatur TG unter 50 °C, vorzugsweise unter 0 °C und ganz besonders bevorzugt im Bereich von -30 bis -110 °C haben. Blockcopolymere mit flexiblen Blöcken verbessern die Bruchzähigkeit ohne die Biegefestigkeit und das E-Modul der Polymerisate wesentlich zu beeinträchtigen.

Erfindungsgemäß bevorzugt sind Polyester-Polysiloxan-Blockcopolymere gemäß der folgenden allgemeinen Formel I:

(PCL)_{q}-b-(PDMS)ᵣ-b-(PCL)_{q} Formel I

in der
- q: jeweils eine Zahl von 5 bis 40, bevorzugt 10 bis 20, ist und
- r: eine Zahl von 10 bis 100, bevorzugt 30 bis 60, ist.

(PCL)_{q} steht für Polycaprolacton, das aus q Caprolactonmonomeren aufgebaut ist, und (PDMS)ᵣ für Poly(dimethylsiloxan), das aus r Dimethylsiloxanmonomeren aufgebaut ist. Der Buchstabe b steht für block.

Weiter bevorzugt sind Poly(meth)acrylat-Polysiloxan-Blockcopolymere, die als A-Block einen Polymethylmethacrylat-Rest und als B-Block einen Polysiloxan-Rest enthalten, wobei der Polysiloxan-Rest vorzugsweise wie oben definiert und ganz besonders bevorzugt ein Poly(dimethylsiloxan)-Rest ist.

Besonders bevorzugt sind die ABA-Triblockcopolymere PCL-b-PDMS-b-PCL und PMMA-b-PDMS-b-PMMA mit einem Molverhältnis A:B von 0,1 bis 5 und mit einer Molmasse von bevorzugt 3 bis 25 kDa, besonders bevorzugt 4 bis 20 kDa und ganz besonders bevorzugt 5 bis 10 kDa. Ein bevorzugtes Blockcopolymer ist PCL-b-PDMS-b-PCL, wobei die PDMS-Blöcke eine Molmasse von ca. 3200 g/mol und die PCL-Blöcke eine Molmasse von jeweils ca. 1600 g/mol aufweisen. In einer bevorzugten Ausführungsform beträgt die Molmasse der A-Blöcke jeweils 40-60% und vorzugsweise 50% der Molmasse des B Blockes. In einer weiteren bevorzugten Ausführungsform beträgt die Kettenlänge der A-Blöcke jeweils 40-65% und vorzugsweise 45-60% bis der Kettenlänge des B-Blocks.

Vorzugsweise wird die Zusammensetzung der Blockcopolymere so gewählt, dass diese 60:40 bis 40:60 Gew.-% A-Blöcke und 40:60 bis 60:40 Gew.-% des B-Blocks enthalten, bezogen auf die Gesamtmasse des Blockcopolymers.

Das oder die Blockcopolymere werden vorzugsweise in einer Menge von 0,5 bis 12 Gew.-%, besonders bevorzugt in einer Menge von 1 bis 10 Gew.-% und ganz besonders bevorzugt 2 bis 8 Gew.-% eingesetzt, bezogen auf das Gesamtgewicht des Dentalwerkstoffs.

Es wurde gefunden, dass die erfindungsgemäß verwendeten Blockcopolymere die Bruchzähigkeit der Polymernetzwerke deutlich verbessern und außerdem eine Opakierung der Werkstoffe bewirken. Dadurch kann der Gehalt der zur Opakierung verwendeten Pigmente reduziert werden. Außerdem bewirken sie nur eine relativ geringe Viskositätserhöhung. Ein weiterer Vorteil der erfindungsgemäß verwendeten Blockcopolymere ist, dass sie sich leicht mit den übrigen Komponenten der Werkstoffe homogen mischen lassen, während das homogene Dispergieren von Kern-Schale-Polymerpartikeln deutlich aufwendiger ist. Zudem neigen Partikel zur Sedimentation, so dass Zusammensetzungen auf der Basis von Kern-Schale-Partikeln weniger stabil sind. Die erfindungsgemäß verwendeten Blockcopolymere lassen sich demgegenüber gut in Harzmischungen einarbeiten, so dass eine Anpassung der Werkstoffe an die geplante Anwendung und eine Einstellung der gewünschten Bruchzähigkeit und Brucharbeit problemlos möglich ist.

Die erfindungsgemäßen Dentalwerkstoffe enthalten als **Komponente (a)** vorzugsweise ein monofunktionelles, nicht aromatisches alicyclisches, bicyclisches oder tricyclisches (Meth)acrylat, wobei Acrylate besonders bevorzugt sind.

Bevorzugte alicyclische Meth(acrylate) sind Verbindungen der folgenden Formel, 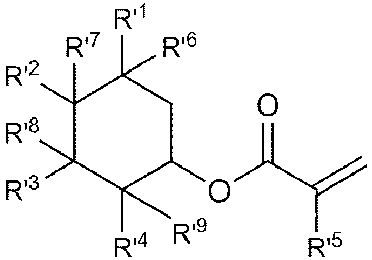 in der
- R'¹ bis R'⁴: unabhängig voneinander ausgewählt sind aus der Gruppe bestehend aus H und C₁-C₆-Alkyl, vorzugsweise H und C₁-C₄-Alkyl, besonders bevorzugt H, Methyl, Ethyl und Isopropyl,
- R'⁵: H oder Methyl ist
- R'⁶ bis R'⁹: unabhängig voneinander ausgewählt ist aus H und C₁-C₃-Alkyl, wobei H besonders bevorzugt ist.

Besonders bevorzugte Beispiele für eine alicyclische Komponenten (a) sind 4-tert-Butylcyclohexyl(meth)acrylat, 2-Isopropyl-5-methylcyclohexyl(meth)acrylat, 3,3,5-Trimethylcyclohexyl(meth)acrylat und Cyclohexylmethacrylat.

Nicht aromatische bi- und tricyclische (Meth)acrylate und insbesondere Acrylate sind ganz besonders bevorzugt. Bevorzugte Beispiele hierfür sind Verbindungen der Formel II: 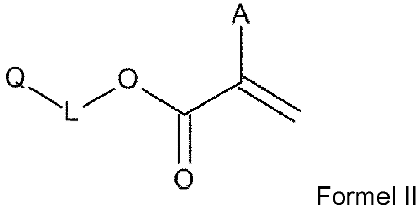 in der
- A: H oder Methyl ist,
- L: eine chemische Bindung, C₁-C₃-Alkylen oder -(CR¹R²-CR³R⁴-O)ₘ- ist, vorzugsweise eine chemische Bindung oder -CH₂-, wobei R¹ bis R⁴ unabhängig voneinander jeweils H oder eine C₁-C₃-Alkylgruppe sind, vorzugsweise H, und m eine ganze Zahl von 2 bis 10, vorzugsweise 2 ist, und
- Q: ein Isobornyl-Derivat oder ein Norbornyl-Derivat ist, vorzugsweise ein unsubstituiertes oder mit einer oder mehreren C₁-C₃-Alkyl substituiertes Isobornyl oder ein unsubstituiertes oder mit einer oder mehreren C₁-C₃-Alkyl substituiertes Norbornyl ist, insbesondere eine der folgenden Gruppen ist, in denen ● den Anknüpfungspunkt zu L symbolisiert:

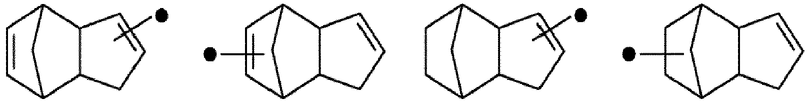

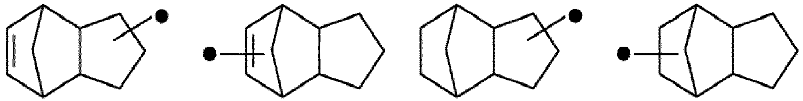

Besonders bevorzugt ist die Verbindung der Formel II ein Mono(meth)acrylat von Tricyclodecanmethanol, Tricyclodecandimethanol oder Dicyclopentadienyl.

Ganz besonders bevorzugt sind die Mono(meth)acrylate von Tricyclodecanmethanol mit den folgenden Formeln in denen A ein H-Atom oder Methyl ist:

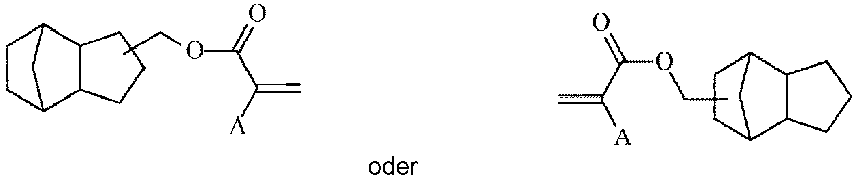

Am meisten bevorzugt ist das Tricyclopentanylacrylat mit der folgenden Formel III:

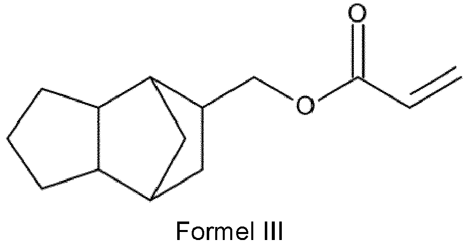

Dieses wird auch als Tricylodecanmethanolmonoacrylat (TCDMA) bezeichnet.

Es können auch Isomerengemische von Verbindungen der Formeln II/III verwendet werden, d.h. die -CH₂-O-Acrylat-Gruppe kann in den Isomeren an unterschiedlichen Positionen mit der tricyclischen Gruppe verknüpft sein.

Die erfindungsgemäßen Dentalwerkstoffe enthalten als **Komponente (b)** vorzugsweise mindestens ein polyfunktionelles Urethanacrylat oder vorzugsweise Urethanmethacrylat, besonders bevorzugt ein Urethandimethacrylat.

Unter polyfunktionellen radikalisch polymerisierbaren Verbindungen werden Verbindungen mit zwei oder mehr, vorzugsweise 2 bis 4 und insbesondere 2 radikalisch polymerisierbaren Gruppen verstanden. Monofunktionelle Monomere weisen dementsprechend nur eine radikalisch polymerisierbare Gruppe auf. Polyfunktionelle Monomere haben vernetzende Eigenschaften und werden daher auch als Vernetzermonomere bezeichnet.

Bevorzugte difunktionelle Urethandimethacrylate (b) sind Urethane aus 2-(Hydroxymethyl)acrylsäuremethylester und Diisocyanaten, wie 2,2,4-Trimethylhexamethylendiisocyanat oder Isophorondiisocyanat, Tetramethylxylylendiurethanethylenglykoldi-(meth)acrylat bzw. Tetramethylxylylendiurethan-2-methylethylenglykoldi(meth)acrylat (V380) und ganz besonders bevorzugt UDMA (ein Additionsprodukt aus 2-Hydroxyethylmethacrylat und 2,2,4-Trimethylhexamethylen-1,6-diisocyanat). Die Komponente (b) ist wie die Komponente (a) ein Monomer. Die erfindungsgemäßen Dentalwerkstoffe enthalten vorzugsweise keine radikalisch polymerisierbaren Oligomere, insbesondere keine oligomeren Urethandimethacrylate.

Das Monomer Tetramethylxylylendiurethanethylenglykoldi(meth)acrylat bzw. Tetramethylxylylendiurethan-2-methylethylenglykoldiurethandi(meth)acrylat (V380) weist die folgende Formel auf:

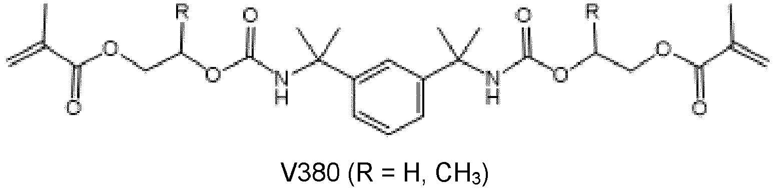

In der gezeigten Formel sind die Reste R unabhängig voneinander jeweils H oder CH₃, wobei die Reste die gleiche Bedeutung oder unterschiedliche Bedeutungen haben können. Vorzugsweise wird eine Mischung eingesetzt, die Moleküle, in denen beide Reste H sind, Moleküle, in denen beide Reste CH₃ sind, und Moleküle enthält, in denen ein Rest H und der andere Rest CH₃ ist, wobei das Verhältnis von H zu CH₃ vorzugsweise 7:3 ist. Eine solche Mischung ist beispielsweise durch Reaktion von 1,3-Bis(1-isocyanato-1-methylethyl)benzol mit 2-Hydroxypropylmethacrylat und 2-Hydroxyethylmethacrylat erhältlich.

Die erfindungsgemäßen Werkstoffe können zwei oder mehr unterschiedliche polyfunktionelle radikalisch polymerisierbare (Meth)acrylate (b) enthalten, besonders bevorzugt mindestens ein polyfunktionelles Urethan(meth)acrylat und ggf. ein weiteres polyfunktionelles (Meth)acrylat.

Bevorzugte weitere di- und polyfunktionelle (Meth)acrylate sind Bisphenol-A-dimethacrylat, Bis-GMA (ein Additionsprodukt aus Methacrylsäure und Bisphenol-A-diglycidylether), ethoxy- oder propoxylierte Bisphenol-A-dimethacrylate, wie z.B. das Bisphenol-A-dimethacrylat SR-348c (Sartomer) mit 3 Ethoxygruppen oder 2,2-Bis[4-(2-methacryloyloxypropoxy)phenyl]propan, Di-, Tri- oder Tetraethylenglycoldimethacrylat, Trimethylolpropantrimethacrylat, Pentaerythrittetramethacrylat sowie Glycerindi- und -trimethacrylat, 1,4-Butandioldimethacrylat, Diol-(Meth)acrylat-Verbindungen, insbesondere 1,10-Decandioldimethacrylat (D₃MA), Bis(methacryloyloxy-methyl)tricyclo-[5.2.1.0^{2,6}]decan (DCP), Polyethylenglycol- oder Polypropylenglycoldimethacrylate, wie z.B. Polyethylenglycol-200-dimethacrylat oder Polyethylenglycol-400-dimethacrylat (PEG-200- oder PEG-400-DMA) oder 1,12-Dodecandioldimethacrylat. Bevorzugte weitere polyfunktionelle (Meth)acrylate sind DecandiolDimethacrylat, Ethylenglykoldimethacrylat (EGDMA) und Butan-1,4-dioldimethacrylat (BDDMA).

Die erfindungsgemäßen Werkstoffe können bis zu 10 Gew.-%, vorzugsweise bis zu 5 Gew.-% weitere polyfunktionelle (Meth)acrylate enthalten. Besonders bevorzugt sind Werkstoffe, die als Komponente (b) ausschließlich ein oder mehrere Urethandi(meth)acrylate und keine weiteren Di(meth)acrylate enthalten.

Vorzugsweise beträgt die Glasübergangstemperatur Tg von Homopolymeren der Komponente (a) 25° oder mehr und die von Homopolymeren der Komponente (b) 110°C oder mehr. Es ist ferner bevorzugt, dass die Differenz der Glasübergangstemperaturen von Homopolymeren der Komponenten (b) und (a) mindestens 40°C beträgt. Glasübergangstemperaturen können gemäß ISO 11357-1:2016 bestimmt werden.

In einer besonders bevorzugten Ausführungsform ist die Komponente (a) ein Acrylat und die Komponente (b) ein Methacrylat. Es ist weiter bevorzugt, dass der erfindungsgemäße Werkstoff ausschließlich Acrylate als Komponente (a) und ausschließlich Methacrylate als Komponente (b) enthält.

Die erfindungsgemäßen Dentalwerkstoffe enthalten als **nanopartikulären oder mikrofeinen Füllstoff (d)** vorzugsweise einen anorganischen partikulären Füllstoff. Bevorzugte anorganische Füllstoffe sind Oxide, wie SiO₂, ZrO₂ und TiO₂ oder Mischoxide aus SiO₂, ZrO₂, ZnO und/oder TiO₂, besonders bevorzugt pyrogene Kieselsäure oder Fällungskieselsäure. Die Füllstoffe weisen eine Primärpartikelgröße von 10 bis 350 nm, vorzugsweise von 10 bis 200 nm und ganz besonders bevorzugt von 20 bis 100 nm auf. Besonders bevorzugte Füllstoffe sind Mischoxide aus SiO₂ und ZrO₂, Fällungskieselsäure oder am meisten bevorzugt pyrogene Kieselsäure.

In einer bevorzugten Ausführungsform weisen die Füllstoffe eine mittlere Primärpartikelgröße (d₅₀) von 50 - 350 nm, insbesondere 100 - 300 nm und besonders bevorzugt 150 - 250 nm auf, gemessen mittels Dynamischer Lichtstreuung (DLS).

Der oder die Füllstoffe (d) wirken als Rheologiemodifikatoren, dienen zur Erhöhung der Härte, des E-Moduls und zur Verbesserung der Abrasionsbeständigkeit. Füllstoffe mit geringer Partikelgröße haben eine größere Verdickungswirkung als Füllstoffe mit größeren Partikeln.

Erfindungsgemäß sind Füllstoffe bevorzugt, die eine BET Oberfläche von 50 bis 300 m²/g aufweisen, besonders bevorzugt 100 bis 200 m²/g und ganz besonders bevorzugt 120 bis 180 m²/g. Es wurde gefunden, dass Füllstoffe mit einer Oberfläche in diesem Bereich bei der gegebenen Partikelgröße einen guten Kompromiss zwischen einer Verbesserung der mechanischen Eigenschaften und einer Erhöhung der Verdickungswirkung erzielen.

Die Bestimmung der BET-Oberfläche erfolgt gemäß DIN ISO 9277 mit einem NOVA 2000e BET Bestimmungsgerät (Quantachrome jetzt Anton Paar / Graz Österreich). Als Adsorbat wird Stickstoff mit einer Temperatur von etwa 77 K benutzt. Das gasförmige Adsorptiv wird dem auf konstanter Temperatur gehaltenen Probenbehälter zugeführt. Die aufgenommenen Adsorbatmengen werden im Gleichgewicht mit dem Gasdruck p des Adsorptivs gemessen und gegen den Relativdruck p/p0 als Adsorptionsisotherme aufgetragen. Daraus kann die spezifische Oberfläche und die Porenvolumenverteilung bestimmt werden. Die Probenaufarbeitung erfolgt durch Ausheizen bzw. Trocknen bei 250 °C für 1 bzw. 3 h. Es wird eine 9 mm-Messzelle verwendet.

In einer bevorzugten Ausführungsform enthalten die erfindungsgemäßen Dentalwerkstoffe eine Mischung von zwei oder mehreren Füllstoffen, insbesondere von zwei oder mehreren Füllstoffen mit unterschiedlichen Partikelgrößen. Es wurde gefunden, dass durch die Verwendung solcher Füllstoffmischungen die Viskosität der Werkstoffe nicht übermäßig erhöht wird und die Zusammensetzungen daher mit generativen Verfahren, wie z.B. mit Hilfe der Stereolithographie, gut verarbeitbar sind. Der Gesamtgehalt an Füllstoffen liegt vorzugsweise in einem Bereich von 0,1 bis 20 Gew.-%, besonders bevorzugt von 2 bis 10 Gew.-%.

Zur Verbesserung des Verbundes zwischen den Füllstoffpartikeln und der vernetzten Polymerisationsmatrix können SiO₂-basierende Füllstoffe mit methacrylat-funktionalisierten Silanen oberflächenmodifiziert werden. Ein bevorzugtes Beispiel für solche Silane ist 3-Methacryloyloxypropyltrimethoxysilan. Es ist weiter bevorzugt, dass Füllstoffe, die mit methacrylat-funktionalisierten Silanen, insbesondere mit 3-Methacryloyloxypropyltrimethoxysilan, oberflächenmodifiziert sind, einen Kohlenstoffgehalt von 3 bis 8 Gew.-%, insbesondere 4,5 bis 6,5 Gew.-%, bestimmt nach ISO 3262-20, aufweisen.

Zur Oberflächenmodifizierung von nichtsilikatischen Füllstoffen wie ZrO₂ oder TiO₂ können auch funktionalisierte saure Phosphate, wie z.B. 10-Methacryloyloxydecyldihydrogenphosphat eingesetzt werden.

Wenn nicht anders angegeben, handelt es sich bei allen Partikelgrößen hierin um gewichtsmittlere Partikelgrößen, wobei die Partikelgrößenbestimmung im Bereich von 0,1 µm bis 1000 µm mittels statischer Lichtstreuung erfolgt, vorzugsweise mit einem statischen Laserstreuungs-Partikelgrößen-Analysator LA-960 (Horiba, Japan). Hierbei werden als Lichtquellen eine Laser-Diode mit einer Wellenlänge von 655 nm und eine LED mit einer Wellenlänge von 405 nm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine 0,1 bis 0,5%ige wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320.

Partikelgrößen kleiner als 0,1 µm werden vorzugsweise mittels Dynamischer Lichtstreuung (DLS) bestimmt. Dafür wird 1 g des zu bestimmenden Füllstoffs in 50 g Wasser aufgenommen und es werden 3 ml 20%-ige Natriumpolyphosphat-Lösung hinzugegeben. Die Messung der Partikelgröße im Bereich von 5 nm bis 0,1 µm erfolgt vorzugsweise durch Dynamische Lichtstreuung (DLS) von wässrigen Partikeldispersionen, vorzugsweise mit einem Korngrößenmessgerät Nanotrac Flex (Microtrac Retsch GmbH / Haan, Deutschland) mit einem He-Ne-Laser mit einer Wellenlänge von 780 nm (Intensität 3 mW), bei einem Messwinkel von 180° bei 25°C. Die Berechnung der Ergebnisse erfolgt über die Auswertung der Autokorrelationsfunktion.

Die erfindungsgemäßen Werkstoffe enthalten vorzugsweise maximal 5 Gew.-%, insbesondere maximal 3 Gew.-%, besonders bevorzugt maximal 0,3 Gew.-% und weiter bevorzugt keine Füllstoff-Teilchen, wie z.B. Primärpartikel, Agglomerate und Aggregate, mit einer mittleren Partikelgröße von mehr als 350 nm. Gröbere Füllstoffe, wie beispielsweise Glaspulver, werden herkömmlicherweise eingesetzt, um Werkstoffe mit guten mechanischen Eigenschaften zu erhalten. Sie werden insbesondere zur Herstellung von Ersatzzähnen verwendet, weil diese beim Kauen hohen Belastungen ausgesetzt sind. Grobe Füllstoffe eignen sich aber nicht zur Herstellung von Prothesenteilen, weil ihre Verwendung zu Problemen bei der Verarbeitung der Werkstoffe sorgt, z.B. wegen ihrer Neigung zur Sedimentation und einer erhöhten Anfälligkeit für Verfärbungen. Feinteilige Füllstoffe haben andererseits den Nachteil, dass sie aufgrund ihrer höheren Verdickungswirkung nur in relativ geringen Mengen einsetzt werden können, was im Hinblick auf die mechanischen Eigenschaften nachteilig ist. Außerdem erschwert eine hohe Viskosität die Verarbeitung mit 3D-Druckern.

Erfindungsgemäß wurde gefunden, dass Dentalwerkstoffe, die sich sowohl zur Herstellung der Prothesenbasis als auch der Ersatzzähne eignen, erhalten werden können, indem nanopartikulären oder mikrofeinen Füllstoff mit einer mittleren Primärpartikelgröße vom 10 nm bis 350 nm und bestimmte ABA-Triblockcopolymere miteinander kombiniert werden.

Erfindungsgemäß bevorzugte **Photoinitiatoren (e)** zur Initiierung der radikalischen Photopolymerisation sind Benzophenon, Benzoin sowie deren Derivate oder α-Diketone oder deren Derivate, wie 9,10-Phenanthrenchinon, 1-Phenyl-propan-1,2-dion, Diacetyl oder 4,4'-Dichlorbenzil. Besonders bevorzugt sind Campherchinon (CQ) und 2,2-Dimethoxy-2-phenyl-acetophenon, insbesondere α-Diketone in Kombination mit Aminen als Reduktionsmittel, wie z.B. 4-(Dimethylamino)-benzoesäureester (EDMAB), N,N-Dimethylaminoethylmethacrylat, N,N-Dimethyl-sym.-xylidin oder Triethanolamin.

Besonders bevorzugte Photoinitiatoren sind weiterhin Norrish-Typ-I-Photoinitiatoren, vor allem Monoacyl- oder Bisacylphosphinoxide, wie z.B. Diphenyl-(2,4,6-trimethylbenzoyl)phenylphosphinoxid (CAS Nr. 75980-60-8), und Monoacyltrialkyl-, Diacyldialkyl- oder Tetraacylgermanium-Verbindungen, wie z.B. Benzoyltrimethylgermanium, Dibenzoyldiethylgermanium, Bis(4-methoxybenzoyl)di-ethylgermanium (MBDEGe), Tetrakis(4-ethoxybenzoyl)germanium oder Tetrakis(4-propoxybenzoyl)germanium, oder Acylzinn-Verbindungen, wie Monoacylstannane, Diacylstannane, Triacylstannane oder Tetraacylstannane, wie z.B. Benzoyltriphenylzinn. Weitere bevorzugte Acylgermanium-Verbindungen werden in der EP 3 150 641 A1 und weitere bevorzugte Acylzinn-Verbindungen in der EP 3 293 215 A1 beschrieben. Dabei lassen sich vorteilhaft auch Mischungen der verschiedenen Photoinitiatoren einsetzen, wie z.B. Bis(4-methoxybenzoyl)diethylgermanium in Kombination mit Campherchinon und 4-Dimethylaminobenzoesäureethylester.

Ganz besonders bevorzugt sind Campherchinon ( CAS Nr. 10373-78-1) in Kombination mit Ethyl-4-(dimethylamino)benzoat (EMBO, CAS Nr. 10287-53-3) sowie Phenylbis(2,4,6-trimethylbenzoyl) phosphinoxid (BAPO, CAS 162881-26-7), Diphenyl(2,4,6-trimethylbenzoyl)phenyl-phosphinoxid (TPO, CAS Nr. 75980-60-8), 2,4,6-Trimethylbenzoyldi-phenylphosphinat (TPO-L, CAS Nr. 84434-11-7), 2-Benzyl-2-(dimethylamino)-4'-morpholinobutyrophenon (Irgacure 369, CAS Nr. 119313-12-1), 1-Butanon-2-(dimethylamino)-2-(4-methylphenyl)methyl-1-4-(4-morpholinyl) phenyl (Irgacure 379, CAS-Nr. 119344-86-4) und ganz besonders Bis(4-methoxy-benzoyl)-diethylgermanium (MBDEGe; Ivocerin), Tetrakis(4-ethoxybenzoyl)germanium oder Tetrakis(4-propoxybenzoyl)germanium. Erfindungsgemäß sind insbesondere solche Photoinitiatoren bevorzugt, die entweder eine nur sehr geringe Eigenfarbe aufweisen oder bei Bestrahlung mit Licht, z.B. im stereolithographischen Druckprozess oder im Nachvergütungsprozess, ihre Farbe verlieren. Die am meisten bevorzugten Photoinitiatoren sind TPO und BAPO.

Die erfindungsgemäßen Werkstoffe können neben den oben genannten Bestandteilen zusätzlich ein oder mehrere **Additive (f)** enthalten, insbesondere Stabilisatoren, Farbmittel, Weichmacher, Thixotropieadditive, Dispergierhilfsmittel, Entschäumer, mikrobizide Wirkstoffe und/oder Treibmittel.

In einer weiteren bevorzugten Ausführungsform enthält der erfindungsgemäße Werkstoff zusätzlich einen thermischen Initiator, vorzugsweise eine Verbindung, die durch Wärme in einen reaktionsfähigen Zustand versetzt werden kann, insbesondere Benzoylperoxid. Ist ein thermischer Initiator im Werkstoff enthalten, kann nach der radikalischen Polymerisation zur Erzielung besserer mechanischer Eigenschaften eine thermische Nachvergütung durchgeführt werden, vorzugsweise bei einer Temperatur von mindestens 80°C, insbesondere mindestens 100°C. Besonders bevorzugt sind Verbindungen, die mindestens eine durch Wärme entfernbare Schutzgruppe umfassen.

Die erfindungsgemäßen Dentalwerkstoffe eignen sich besonders zur Herstellung der Prothesenbasis und der Ersatzzähne von dentalen Voll- oder Teilprothesen. Dies bedeutet nicht, dass die zur Herstellung der Prothesenbasis und die zur Herstellung der Ersatzzähne verwendeten Zusammensetzungen absolut identisch sind. Kern der Erfindung ist vielmehr die Bereitstellung eines Werkstoffs, der durch geringfügige Änderungen an die Herstellung von Prothesenbasen oder Ersatzzähnen angepasst werden kann. Die Anpassung kann durch Variation der Art und Menge der verwendeten Komponenten erfolgen. Die Variation der Art der Komponenten erfolgt vorzugsweise innerhalb der definierten Gruppen und insbesondere der bevorzugten Gruppen. Beispielsweise kann ein Monomer (a) ganz oder vorzugsweise teilweise durch ein anderes Monomer (a) ersetzt werden. Gemäß einer bevorzugten Ausführungsform der Erfindung erfolgt die Anpassung durch eine Variation der Mengen der zur Herstellung der Werkstoffe verwendeten Bestandteile, so dass die Werkstoffe zur Herstellung der Prothesenbasis und zur Herstellung der Ersatzzähne dieselben Bestandteile aber in unterschiedlichen Mengenanteilen enthalten. Es ist zum Beispiel möglich, den Anteil der Blockcopolymere zu variieren oder das Verhältnis zwischen den Komponenten (a) und (b) anzupassen. Die Verwendung anderer oder zusätzlieher Bestandteile ist in geringem Umfang möglich, z.B. können alternative Monomere vom Typ der Komponente (b) in einer Menge von bis zu 10 Gew.-% zugegeben werden. Beispielweise sind Werkstoffe zur Herstellung der Prothesenbasis zahnfleischfarben und Werkstoffe zur Herstellung von Ersatzzähnen zahnfarben eingefärbt, so dass unterschiedliche Farbmittel, insbesondere Pigmente, eingesetzt werden. Im einfachsten Fall erfolgt die Anpassung ausschließlich durch eine entsprechende Einfärbung der Materialien.

Die erfindungsgemäßen Werkstoffe enthalten vorzugweise mindestens ein (1) und vorzugsweise 2 bis 5 Farbmittel. Farbmittel werden vorzugsweise in einer Konzentration von 0,0001 bis 0,5 Gew.-% eingesetzt. Erfindungsgemäß bevorzugte Farbmittel sind organische Farbstoffe und Pigmente, insbesondere Azofarbstoffe, Carbonylfarbstoffe, Cyaninfarbstoffe, Azomethine und Methine, Phthalocyanine und Dioxazine. Besonders bevorzugt sind Farbstoffe, die in den erfindungsgemäßen Werkstoffen löslich sind, insbesondere Azofarbstoffe. Ganz besonders bevorzugt sind organische und insbesondere anorganische Pigmente, die sich gut in den erfindungsgemäßen Dentalwerkstoffen dispergieren lassen. Bevorzugte anorganische Pigmente sind Metalloxide oder Hydroxide, wie z.B. Titandioxid oder ZnO als Weisspigmente, Eisenoxid (Fe₂O₃) als Rotpigment oder Eisenhydroxid (FeOOH) als Gelbpigment. Bevorzugte organische Pigmente sind Azopigmente, wie z.B. Monoazogelb- und Orangepigmente, Disazopigmente oder β-Naphthol-Pigmente, und Nichtazo- oder polycyclische Pigmente, wie z.B. Phthalocyanin-, Chinacridon-, Perylen- und Flavanthron-Pigmente. Besonders bevorzugt sind Azopigmente und Nichtazopigmente. Die am meisten bevorzugten Pigmente sowohl zur Einfärbung der Prothesenbasis und anderer zahnfleischfarbener Prothesenbestandteile als auch der Ersatzzähne sind Titanoxid-Pigmente, Eisenoxide und Azopigmente.

Die erfindungsgemäßen Dentalwerkstoffe können ein oder mehrere Thixotropieadditive enthalten. Diese Additive bewirken eine Verdickung der Werkstoffe und können so beispielsweise ein Sedimentieren der Füllstoffe verhindern. Insbesondere füllstoffhaltige Werkstoffe enthalten daher vorzugweise mindestens ein Thixotropieadditiv. Bevorzugte Thixotropieadditive sind OH-Gruppen-haltige Polymere, wie z.B. Cellulosederivate, und anorganische Stoffe, wie z.B. Schichtsilicate. Um die Viskosität der Werkstoffe nicht zu stark zu erhöhen, enthalten die erfindungsgemäßen Dentalwerkstoffe vorzugsweise nur 0 bis 3,0 Gew.-%, besonders bevorzugt 0 bis 2,0 Gew.-% und ganz besonders bevorzugt kein Thixotropieadditiv, bezogen auf das Gesamtgewicht des Werkstoffs. Bestimmte Füllstoffe, wie z.B. hochdisperses SiO₂, d.h. SiO₂ mit geringer Primärteilchengröße (< 20 nm) und großer Oberfläche (> 100 m²), haben eine thixotropierende Wirkung und können Thixotropieadditive ersetzen.

Die rheologischen Eigenschaften der erfindungsgemäßen Dentalwerkstoffe werden an den gewünschten Anwendungszweck angepasst. Materialen zur stereolithographischen Verarbeitung werden vorzugsweise so eingestellt, dass ihre Viskosität im Bereich von 50 mPa·s bis 50 Pa·s, bevorzugt 100 mPa·s bis 10 Pa·s, besonders bevorzugt 100 mPa·s bis 5 Pa·s liegt. Die Viskosität wird bei 25°C mit einem Kegel-Platte-Viskosimeter bestimmt (Scherrate 100/s). Besonders bevorzugt weisen die erfindungsgemäßen Dentalwerkstoffe eine Viskosität < 10 Pa·s und ganz besonders bevorzugt < 5 Pa·s bei 25°C auf. Die Viskosität wird vorzugsweise mit einem Anton Paar Viskosimeter des Typs MCR 302 mit CP25-2 Konus-Platte-Messmittel und einem Messspalt von 53 µm in Rotation bei einer Scherrate von 100/s bestimmt. Aufgrund der geringen Viskosität sind die erfindungsgemäßen Dentalwerkstoffe besonders dazu geeignet, mit Hilfe von generativen Fertigungsverfahren, wie z.B. 3D-Druck oder Stereolithographie, verarbeitet zu werden. Die Verarbeitungstemperatur liegt vorzugsweise in einem Bereich von 10 bis 70°C, besonders bevorzugt 20 bis 30 °C.

Erfindungsgemäß sind Dentalwerkstoffe mit der folgenden Zusammensetzung besonders bevorzugt:
(a) 30 bis 70 Gew.-%, vorzugsweise 30 bis 61 Gew.-% und besonders bevorzugt 40 bis 60 Gew.-% mindestens eines monofunktionellen radikalisch polymerisierbaren (Meth)acrylats,
(b) 20 bis 60 Gew.-%, vorzugsweise 30 bis 55 Gew.-% und besonders bevorzugt 33 bis 55 Gew.-% mindestens eines polyfunktionellen radikalisch polymerisierbaren (Meth)acrylats,
(c) 0,5 bis 16 Gew.-%, vorzugsweise 1 bis 13 Gew.-% und besonders bevorzugt 2 bis 10 Gew.-% mindestens eines ABA-Triblockcopolymers,
(d) 0,2 bis 12 Gew.-%, vorzugsweise 1 bis 10 Gew.-% und besonders bevorzugt 2 bis 8 Gew.-% mindestens eines nanopartikulären oder mikrofeinen Füllstoffs,
(e) 0,1 bis 5,0 Gew.-%, vorzugsweise 0,2 bis 4,0 Gew.-% und besonders bevorzugt 0,3 bis 3,0 Gew.-% mindestens eines Photoinitiators für die radikalische Polymerisation, und
(f) 0 bis 3 Gew.-% Additive.

Wenn nicht anders angegeben, beziehen sich alle Prozentangaben hierin auf die Gesamtmasse des Werkstoffs.

Die Komponente (b) kann 0 bis 35 Gew.-%, vorzugsweise 0 bis 25 Gew.-% und besonders bevorzugt 0 bis 10 Gew.-% eines oder mehrerer weiterer Dimethacrylate enthalten, bezogen auf die Gesamtmasse des Werkstoffs, wobei Dentalwerkstoffe, die keine weiteren radikalisch polymerisierbaren Polymethacrylate enthalten, am meisten bevorzugt sind.

Es wurde überraschenderweise gefunden, dass die spezifische Kombination der genannten Bestandteile Dentalwerkstoffe ergibt, die sich sowohl zur Herstellung von Prothesenbasen und anderen zahnfleischfarbenen Teilen von dentalen Voll- oder Teilprothesen als auch zur Herstellung der Ersatzzähne der Prothesen eignen.

Die erfindungsgemäß verwendeten Triblockcopolymere ergeben in Kombination mit den nanopartikulären oder mikrofeinen Füllstoffen und einer definierten Monomermischung Werkstoffe, die durch Variation der Mengenverhältnisse der Komponenten und eine entsprechende Einfärbung an die unterschiedlichen mechanischen Anforderungen, wie Zähigkeit, Biegefestigkeit und Biegemodul, und ästhetischen Anforderungen, wie Opazität und Einfärbung, von Prothesenbasen und Ersatzzähnen angepasst werden können. Als besonders vorteilhaft erwiesen sich Werkstoffe mit einer Monomermischung, die mindestens ein tricyclisches Acrylat und mindestens ein polyfunktionelles Urethandimethacrylat enthält, besonders bevorzugt eine Mischung mindestens eines Monomers der Formel II oder III und eines Urethandimethacrylats und am meisten bevorzugt eine Mischung von TCDA und UDMA.

Die angepassten Werkstoffe zeichnen sich durch einen ähnlichen Polymerisationsschrumpf, eine vergleichbare Wasseraufnahme und gute Biokompatibilität aus. An den Grenzflächen zwischen Prothesenbasis und Ersatzzähnen wird so eine feste Bindung sowohl dann erreicht, wenn Prothesenbasis und Ersatzzähne nacheinander hergestellt und dann zusammengefügt werden, als auch dann, wenn die Werkstoffe parallel verarbeitet werden, also Prothesenbasis und Ersatzzähne z.B. in einem Inkjet-Druckvorgang gemeinsam hergestellt werden. Dies ist im Hinblick auf generative Herstellungsverfahren ein großer Vorteil und erleichtert die Herstellung dentaler Voll- oder Teilprothesen mit solchen Verfahren erheblich.

Die monofunktionellen Methacrylate und insbesondere Acrylate der Formel II und ganz besonders der Formel III zeichnen sich durch eine hohe Flexibilität, Reaktivität und eine gute Mischbarkeit mit den anderen Komponenten, d.h. insbesondere mit den Monomeren der Komponente (b) und mit den Blockcopolymeren, aus. Es wurde zum Beispiel festgestellt, dass TCDMA mit Monomeren der Komponente (b), insbesondere UDMA, und den Blockcopolymeren gut mischbar ist.

Die erfindungsgemäßen Dentalwerkstoffe zeichnen sich dadurch aus, dass sie eine gute Bruchzähigkeit und Brucharbeit und gleichzeitig eine hohe Biegefestigkeit und ein relativ hohes E-Modul aufweisen, gemessen bei 37°C in Wasser, was oralen Bedingungen entspricht. Die Werkstoffe weisen außerdem vor der Härtung eine hohe Transparenz und eine niedrige Viskosität auf. Für die gewählte Anwendung ist weiterhin vorteilhaft, dass die Dentalwerkstoffe nach der Härtung eine höhere Opazität als vor der Härtung aber eine geringe Eigenfarbe haben.

Werkstoffe zur Herstellung von Prothesenbasen weisen nach der Härtung vorzugsweise eine Bruchzähigkeit Kₘₐₓ von größer 1,0 MPa·m^{1/2}, bevorzugt größer 1,2 MPa·m^{1/2}, besonders bevorzugt größer 1,4 MPa·m^{1/2} sowie eine Brucharbeit FW größer 150 J/m², bevorzugt größer 175 J/m², besonders bevorzugt größer 200 J/m² auf. Die Bestimmung der Bruchzähigkeit Kₘₐₓ und der Brucharbeit FW erfolgt in Anlehnung an ISO 20795-1:2013 im 3-Punkt Biegeversuch bei einer Stützweite von 32 mm. Außerdem weisen die gehärteten Materialen vorzugsweise ein Biegemodul, bestimmt gemäß ISO20795-1:2013, von mindestens 1500 MPa, bevorzugt von 2000 MPa oder mehr, besonders bevorzugt von 2150 MPa oder mehr auf. Es ist weiter bevorzugt, dass die Materialien nach der Härtung eine Biegefestigkeit, bestimmt gemäß ISO20795-1:2013, von 50 MPa oder mehr, vorzugsweise 60 MPa oder mehr und besonders bevorzugt 65 MPa oder mehr aufweisen.

Werkstoffe zur Herstellung von Ersatzzähnen weisen nach der Härtung vorzugsweise eine Bruchzähigkeit Kₘₐₓ von größer 0,8 MPa·m^{1/2}, bevorzugt größer 1,0 MPa·m^{1/2}, besonders bevorzugt größer 1,1 MPa·m^{1/2} sowie eine Brucharbeit FW größer 100 J/m², bevorzugt größer 125 J/m², besonders bevorzugt größer 130 J/m² auf. Außerdem weisen die gehärteten Materialen vorzugsweise ein Biegemodul, bestimmt gemäß ISO20795-1:2013, von mindestens 2200 MPa, bevorzugt von 2400 MPa oder mehr, besonders bevorzugt von 2500 MPa oder mehr auf. Es ist weiter bevorzugt, dass die Materialien nach der Härtung eine Biegefestigkeit, bestimmt gemäß ISO20795-1:2013, von 60 MPa oder mehr, vorzugsweise 65 MPa oder mehr und besonders bevorzugt 70 MPa oder mehr aufweisen.

Zur Bestimmung von Bruchzähigkeit Kₘₐₓ, Brucharbeit FW, Biegefestigkeit und Biegemodul wird abweichend von der ISO20795-1:2013 ein gedruckter Prüfkörper verwendet. Die Herstellung der Prüfkörper erfolgt auf einem PrograPrint PR5 Stereolithographiesystem (Ivoclar / Schaan, Liechtenstein) unter Zuhilfenahme der Software PrograPrint CAM (Ivoclar / Schaan, Liechtenstein). Die Prüfkörper werden mit einer Schichthöhe von 100 µm bei Raumtemperatur (23 °C) mit einem Aufmass von 0,1 mm (x-, y-, z-Richtung) erstellt und nach dem Druckprozess in einem Reinigungsgerät PrograPrint Clean (Ivoclar / Schaan Liechtenstein) für je 2 min bei einer Umdrehungszahl von 850 U/min in den beiden Reinigungsbädern auf der Bauplattform gereinigt. Nach dem Reinigen werden die Prüfkörper mit ölfreier Druckluft abgeblasen und im PrograPrint Cure Nachvergütungsgerät (Ivoclar / Schaan, Liechtenstein) für 90 Sekunden bei einer Wellenlänge von 405 nm bei 100 % Leistung nachvergütet. Dann werden die Prüfkörper mit Nassschleifpapier der Körnung P1000 auf das für die Normprüfungen der ISO20795-1 benötigte Maß geschliffen und nach Vorgabe der Normprüfung eingelagert und geprüft.

Werkstücke, die aus diesen Materialien hergestellt werden, halten in hohem Maße Verformungen ohne Bruch stand.

Die erfindungsgemäßen Werkstoffe weisen nach der Härtung eine gute Biegefestigkeit, ein gutes Biegemodul und eine gute Bruchzähigkeit sowie eine hohe Brucharbeit auf. Formteile, die durch Härtung der erfindungsgemäßen Werkstoffe erhalten werden, haben eine hohe Steifigkeit und setzen einer Verformung einen hohen Widerstand entgegen, ohne zu brechen.

Es wurde festgestellt, dass die vorstehend beschriebenen bevorzugten Zusammensetzungen des erfindungsgemäßen Werkstoffs zu besonders vorteilhaften mechanische Eigenschaften führen, wobei insbesondere das Molverhältnis von Komponente (a) zu Komponente (b) von Bedeutung ist. Für Prothesenbasismaterialien beträgt das Molverhältnis von Komponente (a) zu Komponente (b) vorzugsweise 1,5 bis 2,0 : 1, insbesondere 1,6 bis 1,9 : 1. Für Zahnmaterialien beträgt das Verhältnis vorzugsweise 1,0 - 1,5 : 1, insbesondere 1,1 bis 1,3 : 1. Werden zusätzlich zu diesem bevorzugten Verhältnis ABA-Triblockcopolymere im bevorzugten Bereich verwendet, erhält der Werkstoff eine besonders vorteilhafte Bruchzähigkeit. Darüber hinaus weist der Werkstoff besonders vorteilhafte Modulwerte auf, wenn zusätzlich mindestens ein nanopartikulärer oder mikrofeiner Füllstoff in der bevorzugten Menge verwendet wird. Die Erfindung ist auch auf die Verwendung der vorstehend beschriebenen Dentalwerkstoffe zur Herstellung einer dentalen Prothese oder eines Teils davon gerichtet.

Die erfindungsgemäßen Werkstoffe eignen sich zur Verarbeitung durch 3D-Druck, insbesondere durch Stereolithographie und 3D-Inkjet-Verfaren. Bevorzugte 3D-Inkjet-Verfahren sind Multimaterial-Inkjet-Druckverfahren.

Bei der Stereolithographie werden zunächst eine Prothesenbasis und ein Zahnbogen gedruckt. In die für die Zähne vorgesehenen Kavitäten in der Prothesenbasis wird als Klebeschicht Prothesenbasis- oder Zahnmaterial in nicht polymerisiertem Zustand gegeben. Dann wird der Zahnbogen auf der Prothesenbasis positioniert, bevor mithilfe von Licht das Prothesenbasis- oder Zahnmaterial der Klebeschicht polymerisiert wird, wodurch eine stabile chemische Verbindung zwischen der Klebeschicht und der Prothesenbasis bzw. dem Zahnbogen entsteht. Die Verwendung von Prothesenbasismaterial als Klebeschicht ist besonders bevorzugt.

Vorzugsweise erfolgt die Herstellung dentaler Prothesen mit einem stereolithographischen Verfahren, das die Schritte umfasst:
(1) Herstellen einer Prothesenbasis mit einem 3D-Druckverfahren unter Verwendung eines vorstehend beschriebenen erfindungsgemäßen Dentalwerkstoffs,
(2) gegebenenfalls zumindest teilweise Härtung der in Schritt (1) erhaltenen Prothesenbasis durch radikalische Polymerisation,
(3) Herstellen eines oder mehrerer Ersatzzähne, insbesondere eines Bogens von miteinander verbundenen Zähnen, mit einem 3D-Druckverfahren unter Verwendung eines vorstehend beschriebenen erfindungsgemäßen Dentalwerkstoffs,
(4) gegebenenfalls zumindest teilweise Härtung des oder der in Schritt (3) erhaltenen Ersatzzähne durch radikalische Polymerisation,
(5) gegebenenfalls Auftragen eines Haftmittels auf eine Oberfläche der in Schritt (2) erhaltenen Prothesenbasis und/oder auf eine Oberfläche des oder in Schritt (4) erhaltenen Ersatzzähne,
(6) Fügen der Prothesenbasis mit dem oder den Ersatzzähnen,
(7) Härtung des in Schritt (6) erhaltenen Produkts durch radikalische Polymerisation.
(8) gegebenenfalls thermische Behandlung.

Durch die optionale thermische Behandlung kann zur Verbesserung der mechanischen Eigenschaften vorteilhaft sein und insbesondere in einer vollständigen Härtung resultieren.

In einer Ausführungsform hat die Prothesenbasis einen oder mehrere Hohlräume, in die die Ersatzzähne eingesetzt werden können. Die Oberfläche des oder der Hohlräume ist vorzugsweise nur teilweise ausgehärtet und wird erst nach dem Einsetzen des oder der Ersatzzähne vollständig gehärtet.

Bei Multimaterial-Inkjet-Druckverfahren können das Prothesenbasismaterial und das Zahnmaterial während des Druckverfahrens miteinander verbunden werden, wodurch, anders als bei der Stereolithographie, nach dem Drucken kein zusätzlicher Verfahrensschritt notwendig ist, um die Bauteile aneinanderzufügen. Es wird zunächst eine erste Schicht aus Prothesenbasismaterial gedruckt, bis an einer digital definierten Position, z.B. innerhalb der Schicht oder auch beim Übergang zur nächsten Schicht, das Material gewechselt und das das Druckverfahren mit dem Zahnmaterial fortgesetzt wird. Die unterschiedlichen Materialien werden üblicherweise mit unterschiedlichen Druckköpfen gedruckt. Auf diese Weise entsteht während des Druckverfahrens ein Übergang zwischen den unterschiedlichen Materialien. Die Reihenfolge, in der Prothesenbasismaterial und Zahnmaterial gedruckt werden, kann beliebig hin und her getauscht werden. Aufgrund der sehr ähnlichen Materialeigenschaften vernetzen sich die beiden chemisch sehr ähnlichen, jedoch optisch und gegebenenfalls mechanisch unterschiedlichen, Materialien während des Druckvorgangs zu einem besonders starken Verbund.

Vorzugsweise erfolgt die Herstellung dentaler Prothesen mit einem Multimaterial-Inkjet-Druckverfahren, das die Schritte umfasst:
(1) schichtweises Auftragen einer Schicht, die aus einem Prothesenbasismaterial, einem Zahnmaterial oder einer Kombination davon besteht, mit einem Inkjet-Drucker,
(2) mindestens teilweises Aushärten des aufgetragenen Materials während oder nach, vorzugsweise nach, dem Auftragen der in Schritt (1) gebildeten Schicht, wobei das Aushärten vorzugsweise mittels UV-Licht erfolgt.
(3) Wiederholen der Schritte (1) und (2) um einen Körper mit der gewünschten geometrischen Form und Farbgebung erhält.

Die Erfindung betrifft auch Prothesenbasen, Ersatzzähne, Voll- oder Teilprothesen, die unter Verwendung der erfindungsgemäßen Dentalwerkstoffe hergestellt wurden.

Im Folgenden wird die Erfindung anhand von Ausführungsbeispielen näher erläutert.

### Ausführungsbeispiele

### Beispiel 1

### Herstellung von Dentalwerkstoffen

Die in Tabellen 1 aufgeführten Komponenten wurden in den angegebenen Mengen homogen miteinander gemischt. Dazu wurden alle festen Komponenten (Blockcopolymer oder Kern-Schale-Partikel, Photoinitiator) mit einem Magnetrührer in den Monomeren dispergiert bzw. gelöst, gegebenenfalls auch unter Erwärmen auf 60 °C. Anschließend wurde das Urethandimethacrylat zugegeben und bis zum Erreichen eines homogenen Gemisches weiter gerührt. Die Blockcopolymere ließen sich problemlos in die Mischungen einarbeiten, ohne dass hierfür ein Rotor-Stator-Dispergiersystem oder eine ähnliches System erforderlich gewesen wäre. Anschließend wurde die Mischung mittels eines Speedmixers (Hauschild GmbH in Hamm/Deutschland) entlüftet.

**Tabelle 1: Zusammensetzung von Werkstoffen zur Herstellung von Prothesenbasen und Ersatzzähnen**

| | **Bsp. 1*** | **Bsp. 2*** | **Bsp. 3** | **Bsp. 4*** | **Bsp. 5*** | **Bsp. 6** |
|---|---|---|---|---|---|---|
| **Bestandteil** | % | % | % | % | % | % |
| (a) TCDMA²⁾ | 32,00 | 41,00 | 39,50 | 34,50 | 32,75 | 31,00 |
| (b) UDMA | 59,50 | 50,50 | 48,50 | 64,50 | 61,25 | 58,00 |
| Molverhältnis a:b | 1.15:1 | 1.73:1 | 1.74:1 | 1.14:1 | 1,14;1 | 1,14:1 |
| (d) Aerosil R 711³⁾ | - | - | 4,00 | - | - | 5,00 |
| (c) Blockcopolymer⁴⁾ | 7,50 | 7,50 | 7,00 | - | 5,00 | 5,00 |
| (e) TPO⁵⁾ | 1,00 | 1,00 | 1,00 | 1,00 | 1,00 | 1,00 |
| Bruchzähigkeit [MPa] | 72,4 | 62,0 | 67,8 | 91,6 | 83,8 | 71,4 |
| E-Modul [MPa] | 1776 | 2010 | 2174 | 2561 | 2432 | 2239 |
| Kₘₐₓ [MPa m^{1/2}] | 1,27 | 1,37 | 1,45 | 0,62 | 1,05 | 1,11 |
| Brucharbeit [J/m²] | 223 | 311 | 282 | 45 | 124 | 131 |
| Indikation | Prothesenbasismaterial | | | Zahnmaterial | | |

| | | | | | | |
|---|---|---|---|---|---|---|
| *) Vergleichsbeispiel ²⁾ Tricyclodecanmethanolmonoacrylat der folgenden Formel III ³⁾ Pyrogene Kieselsäure, die gemäß Hersteller (Evonik Operations GmbH) mit methacrylat-funktionalisierten Silanen funktionalisiert ist und einen Kohlenstoffgehalt von 4,5 bis 6,5 Gew.-% aufweist ⁴⁾ PCL-b-PDMS-b-PCL Blockcopolymer, die Molmasse der PDMS-Blöcke ist 3200 g/mol und die Molmasse der PCL-Blöcke 1600 g/mol ⁵⁾ Diphenyl(2,4,6-trimethylbenzoyl)phenyl-phosphinoxid (CAS Nr. 75980-60-8) | | | | | | |

Alle Beispiele wiesen ein E-Modul von mindestens 2000 MPa und eine Biegefestigkeit von mindestens 65 MPa auf. Damit wiesen alle erfindungsgemäßen Materialien die erforderlichen mechanischen Eigenschaften für die Herstellung von Dentalprothesen auf. Darüber hinaus wiesen die erfindungsgemäßen Materialien eine Bruchzähigkeit von mehr als 1 MPa m^{1/2}und eine Brucharbeit von mehr als 100 J/m² auf, was ebenfalls für die Verwendung als Prothesenmaterial vorteilhaft ist. Zahlreiche bekannte Stereolithographie-Prothesenmaterialien, die z.B. nur aus Dimethacrylaten bestehen, weisen schlechtere mechanische Eigenschaften, wie z.B. eine Bruchzähigkeit unter 1 MPa m^{1/2} oder eine Brucharbeit von weniger als 100 J/m².

Es hat sich darüber hinaus gezeigt, dass das Prothesenbasismaterial von Beispiel 3 und das Zahnmaterial von Beispiel 6 besonders geeignet sind, um in additiven Verfahren, wie in der Stereolithographie und in Inkjet-Druckverfahren, verwendet und miteinander verbunden zu werden. Es wurde festgestellt, dass dabei ein besonders starker Verbund zwischen den Werkstoffen erzeugt wird.

## Patentansprüche

1. Radikalisch polymerisierbarer Werkstoff zur Herstellung von Dentalprothesen, der
(a) mindestens ein monofunktionelles radikalisch polymerisierbares (Meth)acrylat,
(b) mindestens ein polyfunktionelles radikalisch polymerisierbares (Meth)acrylat,
(c) mindestens ein Blockcopolymer,
(d) mindestens einen nanopartikulären oder mikrofeinen Füllstoff mit einer mittleren Primärpartikelgröße vom 10 nm bis 350 nm, und
(e) mindestens einen Photoinitiator enthält, **dadurch gekennzeichnet, dass**
das Blockcopolymer (c) ein ABA-Triblockcopolymer ist.

2. Dentalwerkstoff nach Anspruch 1, der als Komponente (c) ein siliziumhaltiges ABA-Triblockcopolymer enthält, wobei vorzugsweise der A-Block ein oligomerer Polyester oder ein Poly(meth)acrylat ist, insbesondere ein oligomeres Polycaprolacton (PCL), ist, und der B-Block vorzugsweise ein oligomeres Polysiloxan, vorzugsweise ein Poly(dimethylsiloxan) (PDMS), ist.

3. Dentalwerkstoff nach Anspruch 2, der als Komponente (c) ein ABA-Triblockcopolymer des Typs PCL-b-PDMS-b-PCL und/oder Polymethylmethacrylat(PMMA)-b-PDMS-b-PMMA, vorzugsweise des Typs PCL-b-PDMS-b-PCL, enthält, wobei das Molverhältnis von A:B im Bereich von 0,1 bis 5 und die Molmasse des ABA-Triblockcopolymers im Bereich von 3 bis 25 kDa, vorzugweise 4 bis 20 kDa und besonders bevorzugt 5 bis 10 kDa liegt.

4. Dentalwerkstoff nach einem der Ansprüche 1 bis 3, wobei die Komponente (c) 60:40 bis 40:60 Gew.-% A-Blöcke und 40:60 bis 60:40 Gew.-% des B-Blocks enthält.

5. Dentalwerkstoff nach einem der Ansprüche 1 bis 4, wobei die Komponente (a) ein nicht aromatisches alicyclisches, bicyclisches oder tricyclisches Methacrylat oder vorzugsweise Acrylat, besonders bevorzugt eine Verbindung der folgenden Strukturformel II enthält: 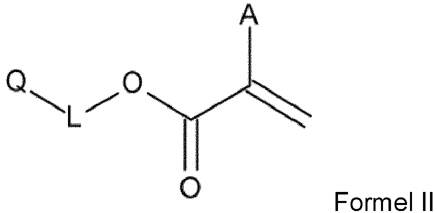 in der
A H oder Methyl ist,
L eine chemische Bindung, C₁-C₃-Alkylen oder -(CR¹R²-CR³R⁴-O)ₘ- ist, vorzugsweise eine chemische Bindung oder -CH₂-, wobei R¹ bis R⁴ unabhängig voneinander jeweils H oder eine C₁-C₃-Alkylgruppe sind, vorzugsweise H, und m eine ganze Zahl von 2 bis 10, vorzugsweise 2 ist, und
Q eine der folgenden Gruppen ist, in denen ● den Anknüpfungspunkt zu L symbolisiert:

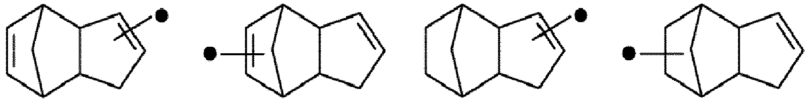

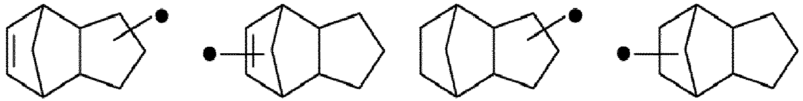

6. Dentalwerkstoff nach Anspruch 5, wobei die Komponente (a) ein Tricyclodecanmethanolmonoacrylat mit der folgenden Strukturformel III enthält:

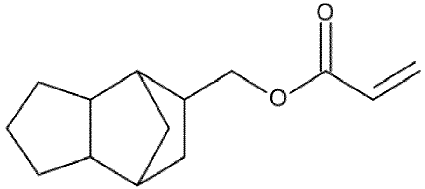

7. Dentalwerkstoff nach einem der Ansprüche 1 bis 6, wobei der Komponente (b) ein polyfunktionelles Urethan(meth)acrylat, vorzugsweise ein Urethanmethacrylat und besonders bevorzugt Urethanmethacrylat (UDMA) enthält.

8. Dentalwerkstoff nach einem der Ansprüche 1 bis 7, wobei die Komponente (d) SiO₂, ZrO₂, TiO₂, ein Mischoxid von SiO₂, ZrO₂, ZnO und/oder TiO₂, vorzugsweise pyrogene Kieselsäure oder Fällungskieselsäure enthält.

9. Dentalwerkstoff nach Anspruch 8, wobei die Komponente (d) eine BET Oberfläche von 50 bis 300 m²/g, vorzugsweise 100 bis 200 m²/g und besonders bevorzugt 120 bis 180 m²/g aufweist.

10. Dentalwerkstoff nach einem der Ansprüche 1 bis 9, der
(a) 30 bis 70 Gew.-%, vorzugsweise 30 bis 61 Gew.-% und besonders bevorzugt 40 bis 60 Gew.-% mindestens eines monofunktionellen radikalisch polymerisierbaren (Meth)acrylats,
(b) 20 bis 60 Gew.-%, vorzugsweise 30 bis 55 Gew.-% und besonders bevorzugt 33 bis 55 Gew.-% mindestens eines polyfunktionellen radikalisch polymerisierbaren (Meth)acrylats,
(c) 0,5 bis 16 Gew.-%, vorzugsweise 1 bis 13 Gew.-% und besonders bevorzugt 2 bis 10 Gew.-% mindestens eines ABA-Triblockcopolymers,
(d) 0,2 bis 12 Gew.-%, vorzugsweise 1 bis 10 Gew.-% und besonders bevorzugt 2 bis 8 Gew.-% mindestens eines nanopartikulären oder mikrofeinen Füllstoffs,
(e) 0,1 bis 5,0 Gew.-%, vorzugsweise 0,2 bis 4,0 Gew.-% und besonders bevorzugt 0,3 bis 3,0 Gew.-% mindestens eines Photoinitiators für die radikalische Polymerisation, und
(f) 0 bis 3 Gew.-% Additive, insbesondere Pigmente, enthält,
jeweils bezogen auf die Gesamtmasse des Werkstoffs.

11. Dentalwerkstoff nach Anspruch 10, der als Komponente (b) 20 bis 60 Gew.-%, vorzugsweise 30 bis 55 Gew.-% und besonders bevorzugt 33 bis 55 Gew.-% mindestens eines Urethandimethacrylats und 0 bis 35 Gew.-%, vorzugsweise 0 bis 25 Gew.-% und besonders bevorzugt 0 bis 10 Gew.-% weiteren Di(meth)acrylate enthält, jeweils bezogen auf die Gesamtmasse des Werkstoffs.

12. Verwendung eines Dentalwerkstoffs nach einem der Ansprüche 1 bis 11 zur Herstellung einer dentalen Prothese oder eines Teils davon.

13. Verwendung nach Anspruch 12 zur Herstellung einer dentalen Prothese durch 3D-Druck, vorzugsweise durch Stereolithografie.

14. Verwendung nach Anspruch 13 zur Herstellung einer dentalen Prothese durch 3D-Inkjet-Verfahren, insbesondere Multimaterial-Inkjet-Druckverfahren.

15. Verfahren zur Herstellung einer dentalen Prothese umfassend die Schritte:
(1) Herstellen einer Prothesenbasis mit einem 3D-Druckverfahren unter Verwendung eines Dentalwerkstoffs gemäß einem der Ansprüche 1 bis 11,
(2) gegebenenfalls zumindest teilweise Härtung der in Schritt (1) erhaltenen Prothesenbasis durch radikalische Polymerisation,
(3) Herstellen eines oder mehrerer Ersatzzähne mit einem 3D-Druckverfahren unter Verwendung eines Dentalwerkstoffs gemäß einem der Ansprüche 1 bis 11,
(4) gegebenenfalls zumindest teilweise Härtung des oder der in Schritt (3) erhaltenen Ersatzzähne durch radikalische Polymerisation,
(5) gegebenenfalls Auftragen eines Haftmittels auf eine Oberfläche der in Schritt (2) erhaltenen Prothesenbasis und/oder auf eine Oberfläche des oder in Schritt (4) erhaltenen Ersatzzähne,
(6) Fügen der Prothesenbasis mit dem oder den Ersatzzähnen,
(7) Härtung des in Schritt (6) erhaltenen Produkts durch radikalische Polymerisation,
(8) gegebenenfalls thermische Behandlung.
